# EUROPÄISCHE PATENTSCHRIFT

(11) **EP 1 365 737 B1**
(45) Veröffentlichungstag und Bekanntmachung des Hinweises auf die Patenterteilung: **20.04.2005**
(21) Anmeldenummer: 01956511.8
(22) Anmeldetag: 05.07.2001
(51) Int. Cl.: C07C 229/26, C07C 69/157, A61K 31/616, A61P 19/02, A61P 25/06

(54) **STABILE SALZE VON O-ACETYLSALICYLSÄURE MIT BASISCHEN AMINOSÄUREN**
STABLE SALTS OF O-ACETYLSALICYCLIC WITH BASIC AMINO ACIDS
SELS STABLES D'ACIDE O-ACETYLSALICYLIQUE AVEC DES ACIDES AMINES BASIQUES

(30) Priorität: 18.07.2000 DE 10034802
(43) Veröffentlichungstag der Anmeldung: 03.12.2003
(73) Patentinhaber: Bayer HealthCare AG, 51368 Leverkusen (DE)
(72) Erfinder: FRANCKOWIAK, Gerhard, 42115 Wuppertal (DE); APPOLT, Hubert, 51515 Kürten (DE); LEIFKER, Gregor, I-20100 Milano (IT); WIRGES, Hans-Peter, 47800 Krefeld (DE); LEDWOCH, Wolfram, 40764 Langenfeld (DE)
(86) Internationale Anmeldenummer: PCT/EP2001/007669
(87) Internationale Veröffentlichungsnummer: WO 2002/005782

(56) Entgegenhaltungen:
- FR-A- 2 115 060
- US-A- 4 265 888
- DATABASE CA [Online] CHEMICAL ABSTRACTS SERVICE, COLUMBUS, OHIO, US; SATO, DAISUKE ET AL: "Acetylsalicylic acid salts" retrieved from STN Database accession no. 80:6964 CA XP002231107 & JP 48 056815 A (GREEN CROSS CORP.) 9. August 1973 (1973-08-09) in der Anmeldung erwähnt
- PATENT ABSTRACTS OF JAPAN vol. 004, no. 186 (C-036), 20. Dezember 1980 (1980-12-20) & JP 55 127345 A (KYOWA HAKKO KOGYO CO LTD), 2. Oktober 1980 (1980-10-02)
- DATABASE CA [Online] CHEMICAL ABSTRACTS SERVICE, COLUMBUS, OHIO, US; LABORATORIOS LIADE S. A.: "Soluble salts of acetylsalicyclic acid" retrieved from STN Database accession no. 81:151809 CA XP002231108 & ES 388 158 A (LABORATORIOS LIADE S. A.) 16. März 1974 (1974-03-16)
- DATABASE CA [Online] CHEMICAL ABSTRACTS SERVICE, COLUMBUS, OHIO, US; KASAFIREK, EVZEN ET AL: "Preparation of lysine acetylsalicylate salt" retrieved from STN Database accession no. 110:219084 CA XP002231109 & CS 247 688 B (CZECH.) 15. Januar 1987 (1987-01-15)
- DATABASE CA [Online] CHEMICAL ABSTRACTS SERVICE, COLUMBUS, OHIO, US; MITSUI TOATSU CHEMICALS, INC., JAPAN: "Purification of aspirin salts" retrieved from STN Database accession no. 97:44312 CA XP002231110 & JP 57 021345 A (MITSUI TOATSU CHEMICALS, INC., JAPAN) 4. Februar 1982 (1982-02-04)

## Beschreibung

Die vorliegende Erfindung betrifft stabile Salze von o-Acetylsalicyclsäure mit basischen Aminosäuren, ein Verfahren zu deren Herstellung sowie deren Verwendung als Arzneimittel.

Die analgetische Wirkung von o-Acetylsalicylsäure (Aspirin®) wird seit langem therapeutisch genutzt. So wird o-Acetylsalicylsäure als Analgetikum, Antipyretikum, Antirheumatikum, sowie als nichtsteroidaler entzündungshemmender Wirkstoff beispielsweise zur Behandlung von Arthritis, Neuralgien und Myalgien eingesetzt.

Allerdings ist o-Acetylsalicylsäure nur begrenzt löslich und somit nur für eine orale Applikation gut geeignet. Es ist aber bekannt (vgl. JP-A-55127345 JP-A-48056815), dass Salze von o-Acetylsalicylsäure mit basischen Aminosäuren für eine parenterale Applikation geeignet sind. Als basische Aminosäuren werden insbesondere L-Lysin, D,L-Lysin oder Arginin eingesetzt. Es kann auch ein gewisser Anteil an Glycin zugesetzt werden.

Salze von o-Acetylsalicylsäure mit basischen Aminosäuren sind in verschiedenen Indikationen seit langem im Einsatz, so zum Beispiel in den vorstehend genannten Indikationen. Die gute Löslichkeit dieser o-Acetylsalicylate stellt hierbei insbesondere bei einer parenteralen Applikation einen Vorteil gegenüber der o-Acetylsalicylsäure dar. Zudem ist bei längerer oraler Verabreichung eine gute Verträglichkeit der o-Acetylsalicylate hervorzuheben.

Ein gewisser Nachteil der o-Acetylsalicylate lag bisher in deren unzureichender Stabilität. Einerseits resultiert daraus eine eingeschränkte Haltbarkeit der aus diesen Salzen hergestellten pharmazeutischen Präparate. Andererseits kann eine gegebenenfalls erforderliche Sterilisierung des Wirkstoffs wegen der unzureichenden thermischen Stabilität dieser Salze nicht über eine Hitzesterilisierung durchgeführt werden, sondern muss auf anderen Wegen wie beispielsweise durch Begasung mit Ethylenoxid erfolgen.

Die geringe Stabilität der o-Acetylsalicylate ist auf eine dem Fachmann bekannte Rückreaktion des Produkts zu o-Acetylsalicylsäure und der entsprechenden Aminosäure zurückzuführen. Die Aminosäure reagiert dann mit der o-Acetylsalicylsäure unter Abspaltung der Acetylgruppe (Amidolyse) und Freisetzung von Salicylsäure. Die Anwesenheit von Salicylsäure in pharmazeutischen Präparaten ist jedoch unerwünscht und deshalb auf einen geringen, akzeptablen Wert zu begrenzen. Es ist bekannt, dass diese Abbaureaktion pH-abhängig ist (F. Moll, Arch. Pharm. 318 (1985), 120-127). Eine Erniedrigung des pH-Werts führt zu einer verstärkten Protonierung der freigesetzten Aminosäure, so dass diese für die Folgereaktion mit der o-Acetylsalicylsäure nicht oder nur noch sehr eingeschränkt zur Verfügung steht. Dadurch wird die Amidolyse und somit die Freisetzung von Salicylsäure zurückgedrängt.

Zur Erhöhung der Stabilität pharmazeutischer Präparate, welche o-Acetylsalicylate enthalten, wurde deshalb in der Vergangenheit die Zugabe "saurer" Stabilisatoren wie beispielsweise Calciumchlorid vorgeschlagen (vgl. US-A-4 265 888). Die Anwesenheit von Ca-Ionen im Produkt ist jedoch für die Behandlung von cardiovaskulären Erkrankungen nicht unbedenklich.

Es wurde ebenfalls postuliert, dass der Feuchtigkeitsgehalt der o-Acetylsalicylatprodukte einen erheblichen Einfluss auf deren Stabilität hat. Ein anderer Weg, die Stabilität der o-Acetylsalicylate zu erhöhen, bestünde daher in der Herabsetzung des Restfeuchtegehalts durch scharfes Trocknen. Intensives Trocknen bei erhöhter Temperatur führt aber wegen der bereits vorstehend erwähnten Instabilität der Salze bei den dafür erforderlichen Temperaturen nur bedingt oder überhaupt nicht zum gewünschten Ziel.

Es war daher die Aufgabe der vorliegenden Erfindung, Zusammensetzungen bereitzustellen, die ein Salz von o-Acetylsalicylsäure mit einer basischen Aminosäure umfassen, die eine erhöhte Stabilität aufweisen und deshalb die Nachteile der bisher bekannten o-Acetylsalicylate hinsichtlich Lagerung und/oder Sterilisierbarkeit nicht aufweisen.

Diese Aufgabe wird gemäß der vorliegenden Erfindung durch eine Zusammensetzung, umfassend ein Salz von o-Acetylsalicylsäure mit einer basischen Aminosäure, gelöst, wobei das Salz bei einer mit einem Malvern 2600D-Gerät unter Standardbedingungen gemessenen Korngrößenverteilung eine mittlere Korngröße oberhalb einer Korngröße von 160 µm und einen Anteil von mehr als 60 % der Teilchen mit einer Korngröße in einem Bereich von 100 bis 200 µm aufweist.

Erfindungsgemäß bevorzugt sind hierbei Zusammensetzungen, in denen das darin enthaltene Salz von o-Acetylsalicylsäure mit einer basischen Aminosäure bei einer mit einem Malvern 2600D-Gerät unter Standardbedingungen gemessenen Korngrößenverteilung eine mittlere Korngröße oberhalb einer Korngröße von 170 µm und einen Anteil von mehr als 70 % der Teilchen mit einer Korngröße in einem Bereich von 100 bis 200 µm aufweist.

Die vorliegende Erfindung wird durch die beiliegenden Zeichnungen näher erläutert.

Es zeigen:
- Fig. 1: eine graphische Darstellung der Korngrößenverteilung des nach Bsp. 1 hergestellten o-Acetylsalicylats im Vergleich zur Korngrößenverteilung eines handelsüblichen o-Acetylsalicylats (Aspisol®)
- Fig. 2: die Integrale der in Fig. 1 dargestellten Kurven der Korngrößenverteilungen für das erfindungsgemäße Bsp. 1 und Aspisol®
- Fig. 3: und 4 Abbildungen von Kristallen eines o-Acetylsalicylats gemäß Bsp. 1 der vorliegenden Erfindung.

- Fig. 5: und 6 Abbildungen von Kristallen von Aspisol®. Mit den gleichen Kristallen wurden die in Fig. 1 und 2 dargestellten Korngrößenanalysen durchgeführt.
- Fig. 7: eine graphische Darstellung der Stabilität bei verschiedenen Temperaturen. Es ist die Veränderung des Gehalts an freier Salicylsäure (in %) in einem o-Acetylsalicylat gemäß Bsp. 3 der vorliegenden Erfindung und in Aspisol® angegeben.

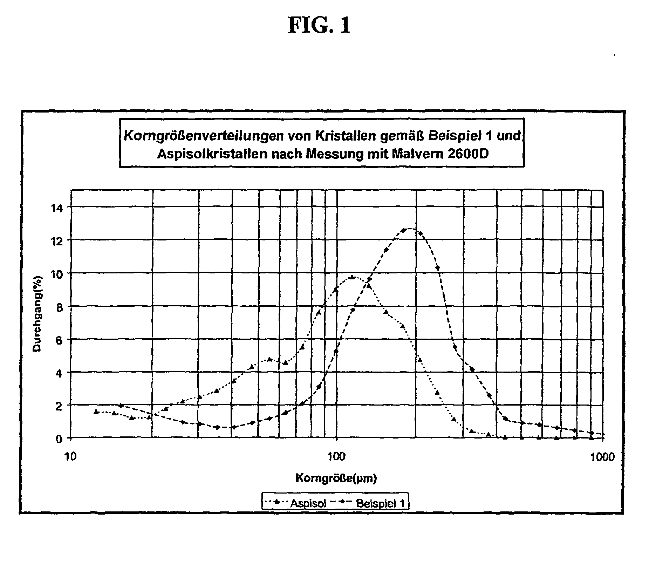

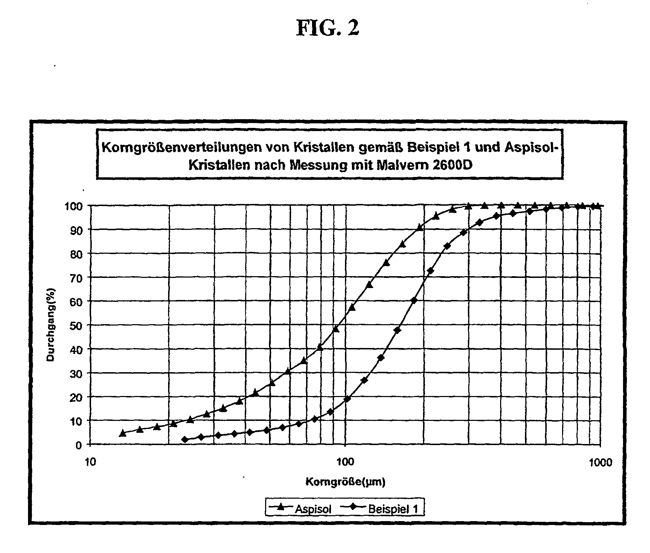

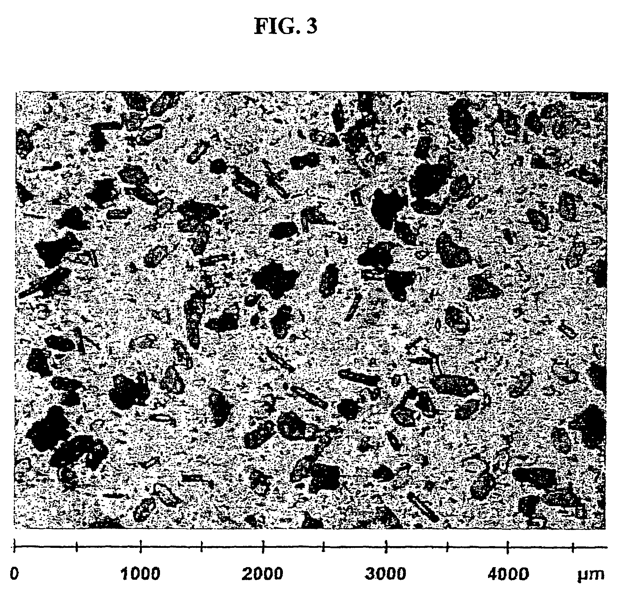

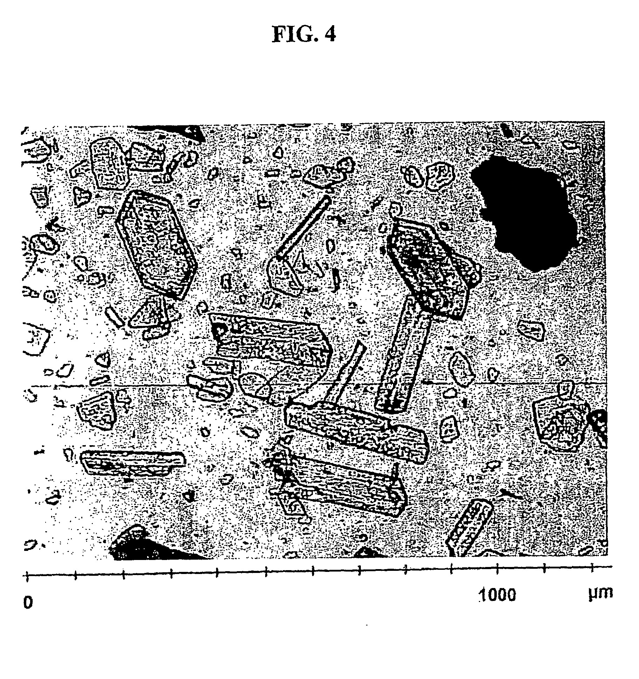

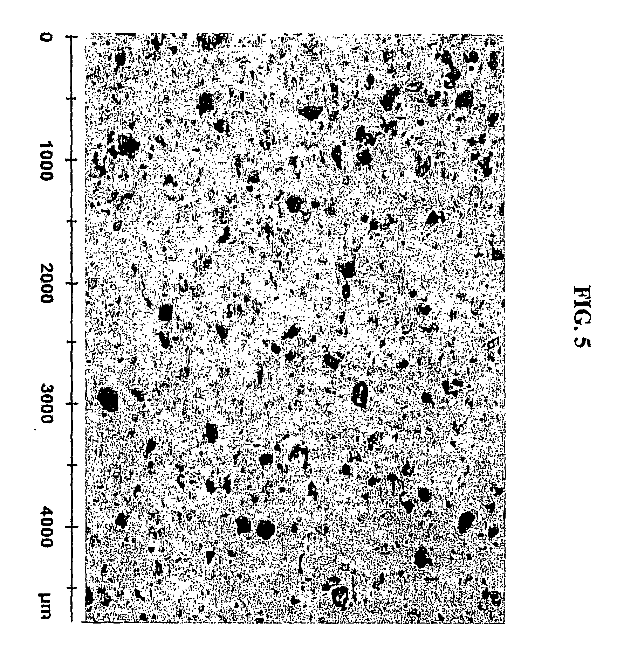

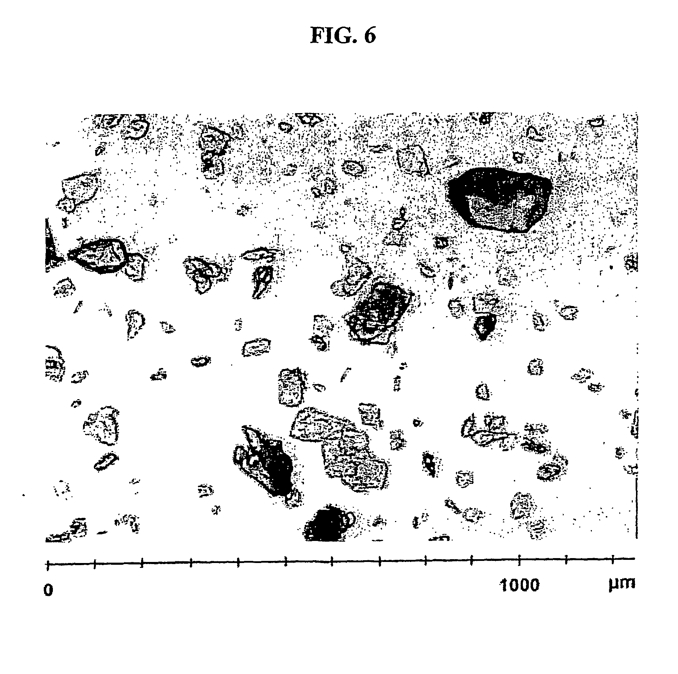

Die erfindungsgemäßen o-Acetylsalicylate unterscheiden sich in ihrer Korngrößenanalyse deutlich und vorteilhaft von den bisher bekannten o-Acetylsalicylaten. Die Verteilung der Korngrößen ist bei den erfindungsgemäßen o-Acetylsalicylaten enger und die mittlere Korngröße zu höheren Partikelabmessungen verschoben (vgl. Fig. 1 und 2). Dies bedeutet, dass die erfindungsgemäßen o-Acetylsalicylate aus größeren und einheitlicher geformten (gewachsenen) Kristallen bestehen. Neben einer engeren Korngrößenverteilung und höheren mittleren Korngröße zeigen die erfindungsgemäßen o-Acetylsalicylate zusätzlich eine gut ausgeprägte Kristallstruktur (vgl. Fig. 3 und 4). Im Vergleich hierzu besitzt das gewerblich erhältliche o-Acetylsalicylat Aspisol® eine deutlich schlechter ausgeprägte Kristallstruktur (vgl. Fig. 5 und 6).

Die vorstehend beschriebenen vorteilhaften Eigenschaften der erfindungsgemäßen o-Acetylsalicylate führen überraschenderweise dazu, dass der Restfeuchtegehalt der erfindungsgemäßen o-Acetylsalicylate außerordentlich niedrig gehalten und somit die vorstehend beschriebene Rückreaktion der o-Acetylsalicylate zu o-Acetylsalicylsäure und der entsprechenden Aminosäure unterdrückt werden kann. Dies ist um so erstaunlicher, da o-Acetylsalicylate an sich als hygroskopisch beschrieben sind. Die erfindungsgemäßen o-Acetylsalicylate weisen aber überraschenderweise eine verminderte Hygroskopie auf. Die erfindungsgemäßen o-Acetylsalicylate sind bei gleicher Temperatur deutlich stabiler als bekannte Acetylsaliclyate mit höherem Restfeuchtegehalt.

Die erfindungsgemäßen Salze weisen einen Restfeuchtegehalt von weniger als 0,4 %, vorzugsweise von weniger als 0,3 % und insbesondere von weniger als 0,15 % Wasser auf.

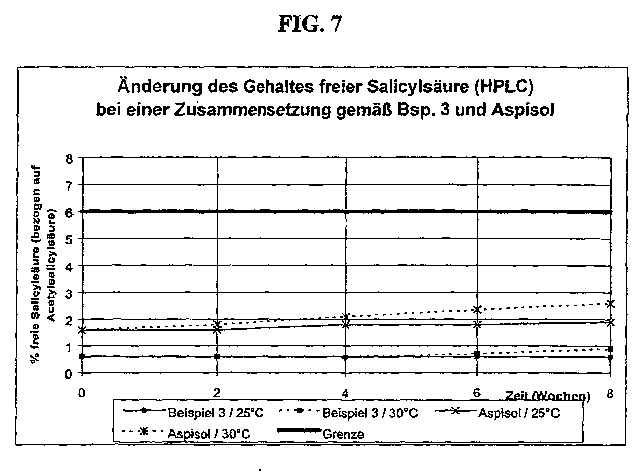

Wie aus Fig. 7 zu entnehmen ist, bleiben erfindungsgemäße o-Acetylsalicylate, welche einen Restfeuchtegehalt von weniger als 0,3 % aufweisen, bei einer Temperatur von 25°C über einen Zeitraum von 8 Wochen hinsichtlich ihres Anteils an freier Salicylsäure nahezu unverändert, während o-Acetylsalicylate mit einem Restfeuchtgehalt von beispielsweise mehr als 0,4 % einem beachtlichen Abbau und Anstieg des Anteils an freier Salicylsäure unterliegen. Somit erweisen sich die erfindungsgemäßen o-Acetylsalicylate bei Raumtemperatur oder unter Kühlung als über lange Zeit stabil.

Die erfindungsgemäßen o-Acetylsalicylate können nach folgendem Verfahren hergestellt werden. Alle Ausgangsverbindungen sind gewerblich erhältlich. Die erfindungsgemäß verwendbaren Aminosäuren können in der L- oder in der D-Konfiguration auftreten oder auch als Gemisch von D- und L-Form. Der Begriff "Aminosäuren" bezeichnet erfindungsgemäß insbesondere die in der Natur vorkommenden α-Aminosäuren, umfasst darüber hinaus aber auch deren Homologe, Isomere und Derivate. Als Beispiel für Isomere können Enantiomere genannt werden. Derivate können beispielsweise mit Schutzgruppen versehene Aminosäuren sein. Als typische Beispiele für basische Aminosäuren seien genannt: Lysin, Arginin, Ornithin, Diaminobuttersäure.

Gemäß der vorliegenden Erfindung werden Lösungen der Reaktionspartner, d.h. von o-Acetylsalicylsäure und der entsprechenden Aminosäure, bei einer Temperatur unterhalb von 30°C, vorzugsweise von 20 bis 25°C, unter Normaldruck möglichst rasch zusammengegeben und zu einer homogenen Phase vermischt. Als Lösungsmittel für die Reaktionspartner kommen Wasser beziehungsweise mit Wasser mischbare organische Lösungsmittel wie beispielsweise Alkohole wie Methanol, Ethanol oder iso-Propanol, insbesondere Ethanol, Ether wie Tetrahydrofuran (THF) oder Ketone wie Aceton in Frage.

Die Reaktionspartner werden in solchen Mengen eingesetzt, dass die basische Aminosäure in leichtem Überschuss vorliegt. Erfindungsgemäß bevorzugt ist ein Verhältnis von o-Acetylsalicylsäure zu Aminosäure von 1:1,05 bis 1:1,5, wobei ein Verhältnis von o-Acetylsalicylsäure zu Aminosäure von 1:1,05 bis 1:1,2 besonders bevorzugt ist.

Gemäß der vorliegenden Erfindung sollte die o-Acetylsalicylsäurelösung einen Gehalt von 1 bis 10 Gew.-%, vorzugsweise 5 bis 10 Gew.-% und insbesondere bevorzugt von 6 bis 8 Gew.-% o-Acetylsalicylsäure aufweisen. Die Lösung der basischen Aminosäure sollte einen Gehalt von 10 bis 40 Gew.-%, vorzugsweise 15 bis 35 Gew.-% und insbesondere bevorzugt von 20 bis 30 Gew.-% Aminosäure aufweisen.

Aus der so hergestellten homogenen Mischung erfolgt anschließend, gegebenenfalls unter Zusatz von Impfkristallen, unter Zugabe eines deutlichen Überschusses an Aceton im Vergleich zu den Reaktionspartnern, beispielsweise eines 20 bis 50 %-igen, vorzugsweise eines 30 bis 40 %-igen Überschusses, die Kristallisation der erfindungsgemäßen Zusammensetzung. Es ist gemäß der vorliegenden Erfindung sehr wichtig, dass die Temperatur der Kristallisationsphase in möglichst engen Grenzen gehalten wird. Die Temperatur darf 40°C nicht übersteigen und sollte vorzugsweise unterhalb von 35°C gehalten werden. Erfindungsgemäß bevorzugt ist eine Temperatur unterhalb von 25°C, insbesondere von 0°C. Als Impfkristalle können Kristalle des gewünschten Produkts, beispielsweise Aspisol®-Kristalle, verwendet werden. Die Kristallisation erfolgt unter Normaldruck.

Ebenso wichtig ist beim erfindungsgemäßen Verfahren das Einhalten einer bestimmten Rührenergie während der Kristallisation. Die homogene Mischung der Ausgangsprodukte darf nur sanft gerührt werden. Die anzulegende Rührenergie sollte nicht größer als 0,1 W pro Liter Reaktionsmedium sein. Erfindungsgemäß bevorzugt ist eine angelegte Rührenergie von 0,04 bis 0,06 W pro Liter Reaktionsmedium. Als Rührer kommen alle herkömmlichen entsprechend regulierbaren Rührgeräte wie beispielsweise ein Rührwerkbehälter mit Stromstörer in Betracht.

Die Lösung sollte zur Kristallisation nicht länger als 20 Stunden unter den vorstehend angegebenen Bedingungen gehalten werden. Erfindungsgemäß bevorzugt ist eine Kristallisationsdauer von weniger als 10 Stunden unter den vorstehend angegebenen Bedingungen, wobei ein Zeitraum von 1 bis 8 Stunden besonders bevorzugt ist.

Auf Wunsch kann die erfindungsgemäße Zusammensetzung auch Glycin enthalten. Die Menge an Glycin ist frei wählbar. Erfindungsgemäß bevorzugt ist ein Anteil von 5 bis 30 Gew.-%, besonders bevorzugt von 5 bis 15 Gew.-% und insbesondere bevorzugt von 10 Gew.-% Glycin, in der Reaktionslösung.

Das Glycin kann gemäß der vorliegenden Erfindung als Lösung in Wasser oder einem mit Wasser mischbaren organischen Lösungsmittel, wobei als organische Lösungsmittel die vorstehend beschriebenen Lösungsmittel verwendet werden können, der Reaktionsmischung aus den Reaktionspartnern zugegeben werden. Glycin verhält sich gegenüber diesen Reaktionspartnem inert. Unter den vorstehend genannten erfindungsgemäßen Bedingungen lassen sich dadurch Kristallisationsvorgänge der beiden Feststoffe (o-Acetylsalicylat und Glycin) aus der homogenen Phase führen (Cokristallisation).

Das Glycin kann gemäß der vorliegenden Erfindung aber auch in Form einer Suspension zu einer bereits kristallisierten Suspension des o-Acetylsalicylats gegeben werden. Die Glycin-Suspension kann auf herkömmliche Weise hergestellt werden. Erfindungsgemäß bevorzugt ist die Herstellung einer Glycin-Suspension aus einem Lösungsmittelgemisch aus Wasser und einem Alkohol wie beispielsweise Ethanol.

Die Art der Zugabe des Glycins hat keinen Einfluss auf die Eigenschaften der erfindungsgemäßen Zusammensetzung. Es ist zu bemerken, dass der Zusatz von Glycin zu den erfindungsgemäßen Zusammensetzungen nicht erforderlich ist. Insbesondere hat die Anwesenheit von Glycin keinen Einfluss auf die Stabilität der erfindungsgemäßen Zusammensetzungen.

Das Kristallisat wird anschließend auf herkömmliche Weise isoliert, beispielsweise durch Filtrieren oder Zentrifugieren. Der Feststoff wird mehrfach mit organischen Lösungsmitteln gewaschen, wobei erfindungsgemäß Alkohole wie beispielsweise Ethanol und/oder Ketone wie Aceton oder Mischungen von Alkoholen oder Ketonen, beispielsweise Mischungen von Ethanol und Aceton, oder die Verwendung verschiedener derartiger Lösungsmittel bevorzugt ist.

Der Feststoff wird anschließend unter vermindertem Druck getrocknet. Hierbei sollte die Temperatur unter 50°C, vorzugsweise unter 40°C und besonders bevorzugt unter 35°C gehalten werden. Es sollte ein Druck von weniger als 50 mbar, vorzugsweise von weniger als 30 mbar an den Feststoff angelegt werden. Die Trocknung kann unter herkömmlichen Bedingungen erfolgen, beispielsweise in einem Trockengerät.

Das erfindungsgemäße Verfahren kann auch vollständig unter sterilen Bedingungen durchgeführt werden. Die hierfür erforderlichen Abweichungen von der obigen Vorgehensweise beispielsweise hinsichtlich Sterilisierung der Ausgangsverbindungen sowie der eingesetzten Apparaturen sind dem Fachmann bekannt.

Die erfindungsgemäßen Zusammensetzungen können als Analgetikum, Antipyretikum, Antirheumatikum, sowie als nichtsteroidale entzündungshemmende Wirkstoffe beispielsweise zur Behandlung von Erkrankungen des rheumatischen Formenkreises, Neuralgien, Myalgien und Migräne eingesetzt werden. Insbesondere können sie aber auch als Thrombozytenaggregationshemmer in der Prävention und Therapie kardio- und cerebrovaskulärer Erkrankungen eingesetzt werden (z.B. bei ischämischen Herzkrankheiten, Schlaganfall (Stroke), stabiler und instabiler Angina pectoris, akutem Myokardinfarkt, Bypass-Operationen, PTCA, Stent-Implantation). Weitere Anwendungsgebiete sind die Stimulierung des Immunsystems bei HIV-Patienten und die Tumorprophylaxe (z.B. Kolon-, Oesophagus- oder Lungenkarzinom), Verlangsamung des kognitiven Verfalls bei dementiellem Syndrom (z.B. Alzheimer-Erkrankung), Hemmung der Gallensteinbildung sowie die Behandlung von diabetischen Erkrankungen.

Zur vorliegenden Erfindung gehören auch pharmazeutische Zubereitungen, die neben nicht-toxischen, inerten pharmazeutisch geeigneten Trägerstoffen die erfindungsgemäßen Zusammensetzungen enthalten, sowie Verfahren zur Herstellung dieser Zubereitungen.

Die erfindungsgemäßen Zusammensetzungen können gegebenenfalls in einem oder mehreren der oben angegebenen Trägerstoffe auch in mikroverkapselter Form vorliegen.

Die erfindungsgemäßen Zusammensetzungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können in einer besonderen Ausführungsform der Erfindung außer den erfindungsgemäßen Zusammensetzungen auch einen oder mehrere weitere pharmazeutische Wirkstoffe in wirksamen Mengen enthalten, insbesondere einen oder mehrere ADP-Rezeptorantagonisten (z.B. Ticlopidin und Clopidogrel), GPIIb/IIIa-Rezeptorantagonisten (z.B. Abciximab, Eptifabitide, Tirofiban, Orofiban, Xemilofiban und Sibrafiban), Phosphodiesterasehemmer (z.B. Dipyridamol), Thrombin-Rezeptorantagonisten (z.B. Hirudin, Hirulog und Argatroban), Faktor Xa-Inhibitoren (z.B. Antistatin, DX-9065 und Pentasaccharide), HMG-CoA-Rezeptorantagonisten (z.B. Simvastatin und Cerivastatin) und/oder Calciumantagonisten (z.B. Nifedipin).

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Der Wirkstoff kann systemisch und/oder lokal wirken. Zu diesem Zweck kann er auf geeignete Weise appliziert werden, wie z.B. oral oder parenteral. Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge, FDT (Fast dissolve Tabletten), Brausetabletten, Kautabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Die topische Applikation in Form von Suppositorien oder von transdermalen systemen (z.B. Pflaster, ETS-Systeme) sowie in Cremes, Salben, gelen, Sprays oder gelöst in organischen oder anorganischen Lösungsmitteln sind weitere Anwendungsmöglichkeiten.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B.Antioxidantien wie Ascorbinsäure), Farbstoffe (z.B. anorganische Pigmente wie Eisenoxide) oder Geschmacks- und / oder Geruchskorrigentien.

### Beispiele

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiel 1: Lysinacetylsalicylat

In einem sterilen und pyrogenfreien Rührwerkbehälter mit Stromstörer gibt man über einen Sterilfilter eine pyrogenfreie Lösung von 9,9 kg o-Acetylsalicylsäure in 120 kg Ethanol. Bei 20 bis 25 °C fügt man innerhalb kurzer Zeit eine sterilfiltrierte pyrogenfreie Lösung von 9,0 kg Lysinhydrat in 26,5 kg pyrogenfreiem Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg sterilfiltriertem Aceton versetzt. Man lässt unter sanftem Rühren bei 0°C eine bis acht Stunden kristallisieren. Das Kristallisat wird unter aseptischen Bedingungen auf einem Filter oder einer Zentrifuge isoliert. Das feuchte Produkt wird auf einem Trennapparat mehrfach mit Ethanol gewaschen. Das feuchte Produkt wird unter aseptischen Bedingungen in einen Trockner überführt und darin bei einem Druck von weniger als 30 mbar bei einer Temperatur von nicht mehr als 40°C getrocknet.

Es wurden 89 bis 94 % des gewünschten Produkts erhalten, das einen Restfeuchtegehalt von 0,10 bis 0,15 % aufwies.

### Beispiel 2: D,L-Lysinacetylsalicylat mit 10 % Glycin

In einem sterilen und pyrogenfreien Rührwerkbehälter mit Stromstörer gibt man über einen Sterilfilter eine pyrogenfreie Lösung von 9,9 kg o-Acetylsalicylsäure in 145 kg Ethanol. Bei 20 bis 25°C fügt man innerhalb kurzer Zeit eine sterilfiltrierte pyrogenfreie Lösung von 9,0 kg D,L-Lysinhydrat und 2,4 kg Glycin in 35 kg pyrogenfreiem Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg sterilfiltriertem Aceton versetzt. Man lässt unter sanftem Rühren bei 0°C eine bis acht Stunden kristallisieren. Das Kristallisat wird unter aseptischen Bedingungen auf einem Filter oder einer Zentrifuge isoliert. Das feuchte Produkt wird auf einem Trennapparat mehrfach nacheinander mit Ethanol und Aceton gewaschen. Das feuchte Produkt wird unter aseptischen Bedingungen in einen Trockner überführt und darin bei einem Druck von weniger als 30 mbar bei einer Temperatur von nicht mehr als 40°C getrocknet.

Es wurden 90 bis 95 % des gewünschten Produkts erhalten, das einen Restfeuchtegehalt von 0,10 bis 0,15 % aufwies.

### Beispiel 3: D,L-Lysinacetylsalicylat mit 10 % Glycin

In einem sterilen und pyrogenfreien Rührwerkbehälter mit Stromstörer gibt man über einen Sterilfilter eine pyrogenfreie Lösung von 9,9 kg o-Acetylsalicylsäure in 120 kg Ethanol. Bei 20 bis 25°C fügt man innerhalb kurzer Zeit eine sterilfiltrierte pyrogenfreie Lösung von 9,0 kg Lysinhydrat in 26,5 kg pyrogenfreiem Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg sterilfiltriertem Aceton versetzt. Man lässt unter sanftem Rühren bei 0°C eine bis acht Stunden kristallisieren. In einem separaten sterilen und pyrogenfreien Rührwerkbehälter wird eine aseptische Suspension von 2,1 kg Glycin in 8 kg pyrogenfreiem Wasser und 25 kg Ethanol hergestellt. Diese wird in die Salicylatsuspension gegeben. Das Kristallgemisch wird unter aseptischen Bedingungen auf einem Filter oder einer Zentrifuge isoliert. Das feuchte Produkt wird auf einem Trennapparat mehrfach mit Ethanol gewaschen. Das feuchte Produkt wird unter aseptischen Bedingungen in einen Trockner überführt und darin bei einem Druck von weniger als 30 mbar bei einer Temperatur von nicht mehr als 40°C getrocknet.

Es wurden 89 bis 94 % des gewünschten Produkts erhalten, das einen Restfeuchtegehalt von 0,10 bis 0,15 % aufwies.

### Bestimmung der Korngrößenverteilung

Die erfindungsgemäßen Zusammensetzungen sowie handelsübliches Aspisol® (von der Firma Bayer AG vertrieben) wurden in einem Malvern 2600 D-Messgerät der Firma Malvern unter folgenden Standardbedingungen untersucht.

Das Messgerät Malvern 2600 besteht aus einem He/Ne-Laser, einer Messküvette mit thermostatisierbarem Vorratsbehälter-System, Fourier-Linsen und Multielement-Detektor. Die gemessenen Lichtintensitäten werden in eine Korngrößenverteilung umgesetzt. Die Ausrichtung von Laser und Linse wird vor jeder Messung manuell eingestellt und das Meßgerät durch eine Leerwertmessung kontrolliert. Die Leerwertimpulse dürfen einen Höchstwert von 20 pro Detektorelement nicht überschreiten.

Die zu untersuchende Probe wird von Hand ca.. 15 s geschüttelt; anschließend wird mit einem Spatel eine Probe entnommen. Die Probenmenge richtet sich nach dem zulässigen Obscuration-Bereich (0.1-0.3) des Messgerätes. Die entnommene Probe wird mit einem üblichen Dispergiermittel wie Baysilon M10® (Firma Bayer AG) schonend (durch Einrühren mit einem Glasstab) in einem Becherglas vordispergiert und anschließend in den Vorratsbehälter des Messgerätes, welcher ebenfalls mit dem Dispergiermittel gefüllt ist, eingefüllt. Das Becherglas wird mit dem Dispergiermittel vollkommen ausgespült, um die repräsentative Probenahme zu gewährleisten.

Die Messung erfolgt mit einer eingestellten Brennweite von 300mm, einer Thermostatisierung von 20°C und einem zulässigen Obscuration-Bereich von 0,1-0,3.

Das Produkt wird nach Ultraschallzeiten von 0, 15 und 60 Sekunden vermessen. Der Ultraschallfinger befindet sich dazu im Vorratsgefäß des Produktumlaufs. Die Suspension wird in einem geschlossenen Kreislauf durch die Messküvette gepumpt.

Die vom Detektor registrierten Signale werden ausgewertet und in die Korngrößenverteilung umgerechnet.

Die so erhaltenen Ergebnisse sind in Fig.1 und 2 dargestellt.

## Patentansprüche

1. Zusammensetzung, umfassend ein Salz von o-Acetylsalicylsäure mit einer basischen Aminosäure, **dadurch gekennzeichnet, dass** das Salz bei einer mit einem Malvem 2600D-Gerät unter Standardbedingungen gemessenen Korngrößenverteilung eine mittlere Korngröße oberhalb einer Korngröße von 160 µm und einen Anteil von mehr als 60 % der Teilchen mit einer Korngröße in einem Bereich von 100 bis 200 µm aufweist.

2. Zusammensetzung nach Anspruch 1, **dadurch gekennzeichnet, dass** das Salz bei einer mit einem Malvem 2600D-Gerät unter Standardbedingungen gemessenen Korngrößenverteilung eine mittlere Korngröße oberhalb einer Korngröße von 170 µm und einen Anteil von mehr als 70 % der Teilchen mit einer Korngröße in einem Bereich von 100 bis 200 µm aufweist.

3. Zusammensetzung nach einem der Ansprüche 1 oder 2, **dadurch gekennzeichnet, dass** die basische Aminosäure aus der Gruppe, bestehend aus Lysin, Arginin, Histidin, Ornithin oder Diaminobuttersäure, ausgewählt ist.

4. Zusammensetzung nach Anspruch 3, **dadurch gekennzeichnet, dass** die basische Aminosäure Lysin ist.

5. Zusammensetzung nach einem der vorstehenden Ansprüche, **dadurch gekennzeichnet, dass** sie zusätzlich einen Anteil von 5 bis 15 Gew.-% Glycin umfasst.

6. Zusammensetzung nach einem der vorstehenden Ansprüche, **dadurch gekennzeichnet, dass** sie zusätzlich einen Anteil von 10 Gew.-% Glycin umfasst.

7. Verfahren zur Herstellung einer Zusammensetzung gemäß einem der Ansprüche 1 bis 6, umfassend das rasche Zusammengeben von Lösungen von o-Acetylsalicylsäure und einer basischen Aminosäure in Wasser oder einem mit Wasser mischbaren organischen Lösungsmittel bei einer Temperatur unterhalb von 30°C unter Normaldruck, so dass die Aminosäure in der Reaktionslösung im leichten Überschuss vorliegt, die Zugabe von Aceton und gegebenenfalls von Impfkristallen bei einer Temperatur unterhalb von 40°C bei Normaldruck, Rühren für nicht länger als 20 Stunden bei einer Rührenergie von nicht mehr als 0,1 W pro Liter Reaktionsmedium, Isolierung des Feststoffs, Waschen mit einem organischen Lösungsmittel und Trocknen bei einer Temperatur unterhalb von 50°C bei einem Druck unterhalb von 50 mbar, sowie gegebenenfalls die Zugabe von 5 bis 30 Gew.-% Glycin als Lösung in Wasser oder in einem mit Wasser mischbaren organischen Lösungsmittel zur Reaktionslösung oder als Suspension zu einer kristallisierten Suspension des o-Acetylsalicylats.

8. Verfahren nach Anspruch 7, **dadurch gekennzeichnet, dass** die Lösungen von o-Acetylsalicylsäure und einer basischen Aminosäure bei einer Temperatur von 20 bis 25°C unter Normaldruck zusammengegeben werden.

9. Verfahren nach Anspruch 7 oder 8, **dadurch gekennzeichnet, dass** das Verhältnis von o-Acetylsalicylsäure zu Aminosäure in der Reaktionslösung von 1:1,05 bis 1:1,2 beträgt.

10. Verfahren nach einem der Ansprüche 7 bis 9, **dadurch gekennzeichnet, dass** ein 30 bis 40 %-iger Überschuss an Aceton zugegeben wird.

11. Verfahren nach einem der Ansprüche 7 bis 10, **dadurch gekennzeichnet, dass** die Temperatur während und nach der Zugabe von Aceton 0°C beträgt.

12. Verfahren nach einem der Ansprüche 7 bis 11, **dadurch gekennzeichnet, dass** nach der Zugabe von Aceton für 1 bis 8 Stunden gerührt wird.

13. Verfahren nach einem der Ansprüche 7 bis 12, **dadurch gekennzeichnet, dass** die Rührenergie 0,04 bis 0,06 W pro Liter Reaktionsmedium beträgt.

14. Verfahren nach einem der Ansprüche 7 bis 13, **dadurch gekennzeichnet, dass** bei einer Temperatur unterhalb von 35°C getrocknet wird.

15. Verfahren nach einem der Ansprüche 7 bis 14, **dadurch gekennzeichnet, dass** bei einem Druck von weniger als 30 mbar getrocknet wird.

16. Verfahren nach einem der Ansprüche 7 bis 15, **dadurch gekennzeichnet, dass** 10 Gew.-% Glycin zugegeben werden.

17. Verfahren nach einem der Ansprüche 7 bis 16, **dadurch gekennzeichnet, dass** das Verfahren unter sterilen Bedingungen durchgeführt wird.

18. Arzneimittel enthaltend mindestens eine Zusammensetzung gemäß einem der vorhergehenden Ansprüche 1 bis 6.

19. Arzneitmittel nach Anspruch 18, **dadurch gekennzeichnet, dass** es einen oder mehrere weitere pharmazeutische Wirkstoffe, insbesondere einen oder mehrere ADP-Rezeptorantagonisten, GPIIb/IIIa-Rezeptorantagonisten, Phosphodiesterasehemmer, Thrombin-Rezeptorantagonisten, Faktor Xa-Inhibitoren, HMG-CoA-Rezeptorantagonisten und/oder Calciumantagonisten, enthält.

20. Verwendung von Zusammensetzungen gemäß einem der vorhergehenden Ansprüche 1 bis 6 zur Herstellung eines Arzneimittels zur Behandlung von Arthritis, Neuralgien, Myalgien und/oder Migräne.

21. Verwendung von Zusammensetzungen gemäß einem der vorhergehenden Ansprüche 1 bis 6 zur Herstellung eines Arzneimittels zur Behandlung von myocardialem Infarkt, Schlaganfall, ischämischen Herzkrankheiten, Angina pectoris, Bypass-Operationen, PTCA und/oder Stent-Implantation.

22. Verwendung von Zusammensetzungen gemäß einem der vorhergehenden Ansprüche 1 bis 6 zur Herstellung einer parenteralen Applikationsform.

23. Verwendung von Zusammensetzungen gemäß einem der vorhergehenden Ansprüche 1 bis 6 zur Herstellung von Injektions- und Infusionszubereitungen in Form von Lösungen, Suspensionen, Emulsionen, Lyophilisaten und sterilen Pulvern.

24. Verwendung von Zusammensetzungen gemäß einem der vorhergehenden Ansprüche 1 bis 6 zur Herstellung eines Arzneimittels zur intravenösen, intraarteriellen, intrakardialen, intraspinalen, intralumbalen, intramuskulären, subcutanen, intracutanen oder intraperitonealen Applikation.

25. Arzneimittel gemäß Anspruch 18, **dadurch gekennzeichnet, dass** es sich um eine parenterale Applikationsform handelt.

26. Arzneimittel gemäß Anspruch 18, **dadurch gekennzeichnet, dass** es sich um eine Injektions- und Infusionszubereitungen in Form von Lösungen, Suspensionen, Emulsionen, Lyophilisaten und sterilen Pulvern.

27. Arzneimittel gemäß Anspruch 18 zur intravenösen, intraarteriellen, intrakardialen, intraspinalen, intralumbalen, intramuskulären, subcutanen, intracutanen oder intraperitonealen Applikation.

## Claims

1. Composition comprising a salt of o-acetylsalicylic acid with a basic amino acid, **characterized in that** the salt has an average particle size above a particle size of 160 µm and a proportion of more than 60% of the particles having a particle size in a range from 100 to 200 µm in a particle size distribution measured using a Malvern 2600D apparatus under standard conditions.

2. Composition according to Claim 1, **characterized in that** the salt has an average particle size above a particle size of 170 µm and a proportion of more than 70% of the particles having a particle size in a range of 100 to 200 µm in a particle size distribution measured using a Malvem 2600D apparatus under standard conditions.

3. Composition according to either of Claims 1 and 2, **characterized in that** the basic amino acid is selected from the group consisting of lysine, arginine, histidine, ornithine or diaminobutyric acid.

4. Composition according to Claim 3, **characterized in that** the basic amino acid is lysine.

5. Composition according to one of the above claims, **characterized in that** it additionally comprises a proportion of 5 to 15% by weight of glycine.

6. Composition according to one of the above claims, **characterized in that** it additionally comprises a proportion of 10% by weight of glycine.

7. Process for the production of a composition according to one of Claims 1 to 6, comprising the rapid combination of solutions of o-acetylsalicylic acid and a basic amino acid in water or a water-miscible organic solvent at a temperature below 30°C under normal pressure, so that the amino acid is present in the reaction solution in a slight excess, the addition of acetone and, if appropriate, of seed crystals at a temperature below 40°C at normal pressure, stirring for not longer than 20 hours at a stirring energy of not more than 0.1 W per litre of reaction medium, isolation of the solid, washing with an organic solvent and drying at a temperature below 50°C at a pressure below 50 mbar, and, if appropriate, the addition of 5 to 30% by weight of glycine as a solution in water or in a water-miscible organic solvent to the reaction solution or as a suspension to a crystallized suspension of the o-acetylsalicylate.

8. Process according to Claim 7, **characterized in that** the solutions of o-acetylsalicylic acid and a basic amino acid are combined at a temperature of 20 to 25°C under normal pressure.

9. Process according to Claim 7 or 8, **characterized in that** the ratio of o-acetylsalicylic acid to amino acid in the reaction solution is from 1:1.05 to 1:1.2.

10. Process according to one of Claims 7 to 9, **characterized in that** a 30 to 40% strength excess of acetone is added.

11. Process according to one of Claims 7 to 10, **characterized in that** the temperature during and after the addition of acetone is 0°C.

12. Process according to one of Claims 7 to 11, **characterized in that** after the addition of acetone the mixture is stirred for 1 to 8 hours.

13. Process according to one of Claims 7 to 12, **characterized in that** the stirring energy is 0.04 to 0.06 W per litre of reaction medium.

14. Process according to one of Claims 7 to 13, **characterized in that** drying is carried out at a temperature below 35°C.

15. Process according to one of Claims 7 to 14, **characterized in that** drying is carried out at a pressure of less than 30 mbar.

16. Process according to one of Claims 7 to 15, **characterized in that** 10% by weight of glycine is added.

17. Process according to one of Claims 7 to 16, **characterized in that** the process is carried out under sterile conditions.

18. Medicament comprising at least one composition according to one of the preceding Claims 1 to 6.

19. Medicament according to Claim 18, **characterized in that** it contains one or more further pharmaceutical active compounds, in particular one or more ADP receptor antagonists, GPIIb/IIIa receptor antagonists, phosphodiesterase inhibitors, thrombin receptor antagonists, factor Xa inhibitors, HMG-CoA receptor antagonists and/or calcium antagonists.

20. Use of compositions according to one of the preceding Claims 1 to 6 for the production of a medicament for the treatment of arthritis, neuralgia, myalgia and/or migraine.

21. Use of compositions according to one of the preceding Claims 1 to 6 for the production of a medicament for the treatment of myocardial infarct, stroke, ischaemic heart diseases, angina pectoris, bypass operations, PTCA and/or stent implantation.

22. Use of compositions according to one of the preceding Claims 1 to 6 for the production of a parenteral administration form.

23. Use of compositions according to one of the preceding Claims 1 to 6 for the production of injection and infusion preparations in the form of solutions, suspensions, emulsions, lyophilizates and sterile powders.

24. Use of compositions according to one of the preceding Claims 1 to 6 for the production of a medicament for intravenous, intraarterial, intracardiac, intraspinal, intralumbar, intramuscular, subcutaneous, intracutaneous or intraperitoneal administration.

25. Medicament according to Claim 18, **characterized in that** it comprises a parenteral administration form.

26. Medicament according to Claim 18, **characterized in that** it comprises injection and infusion preparations in the form of solutions, suspensions, emulsions, lyophilizates and sterile powders.

27. Medicament according to Claim 18 for intravenous, intraarterial, intracardiac, intraspinal, intralumbar, intramuscular, subcutaneous, intracutaneous or intraperitoneal administration.

## Revendications

1. Composition, comprenant un sel d'acide o-acétylsalicylique d'un acide aminé basique, **caractérisée en ce que** le sel représente, pour une répartition de grosseur de grain mesurée dans des conditions normales avec un appareil Malvern 2600D, une grosseur de grain moyenne au-dessus d'une grosseur de grain de 160 µm et une proportion de plus de 60 % des particules avec une grosseur de grain dans une plage de 100 à 200 µm.

2. Composition suivant la revendication 1, **caractérisée en ce que** le sel présente, pour une répartition de grosseur de grain mesurée dans des conditions normales avec un appareil Malvern 2600D, une grosseur de grain moyenne au-dessus d'une grosseur de grain de 170 µm et une proportion de plus de 70 % des particules avec une grosseur de grain dans une plage de 100 à 200 µm.

3. Composition suivant l'une des revendications 1 ou 2, **caractérisée en ce que** l'acide aminé basique est choisi dans le groupe comprenant la lysine, l'arginine, l'histidine, l'ornithine ou l'acide diaminobutyrique.

4. Composition suivant la revendication 3, **caractérisée en ce que** l'acide aminé basique est la lysine.

5. Composition suivant l'une des revendications précédentes, **caractérisée en ce qu'**elle comprend en outre une proportion de 5 à 15 % de glycine.

6. Composition suivant l'une des revendications précédentes, **caractérisée en ce qu'**elle comprend en outre une proportion de 10 % en poids de glycine.

7. Procédé de préparation d'une composition suivant l'une des revendications 1 à 6, comprenant la réunion rapide de solutions d'acide o-acétylsalicylique et d'un acide aminé basique dans l'eau ou dans un solvant organique miscible à l'eau à une température inférieure à 30°C sous pression normale, de manière que l'acide aminé soit présent en léger excès dans la solution réactionnelle, l'addition d'acétone et, le cas échéant, de germes cristallins à une température inférieure à 40°C sous pression normale, l'agitation pendant pas plus de 20 heures avec une énergie d'agitation de pas plus de 0,1 W par litre de milieu réactionnel, l'isolement de la matière solide, le lavage avec un solvant organique et le séchage à une température inférieure à 50°C, à une pression inférieure à 50 mbar, ainsi que le cas échéant l'addition de 5 à 30 % en poids de glycine sous forme de solution dans l'eau ou dans un solvant organique miscible à l'eau à la solution réactionnelle ou sous forme de suspension à une suspension cristallisée du o-acétylsalicylate.

8. Procédé suivant la revendication 7, **caractérisé en ce que** les solutions d'acide o-acétylsalicylique et d'un acide aminé basique sont réunies à une température de 20 à 25°C sous pression normale.

9. Procédé suivant la revendication 7 ou 8, **caractérisé en ce que** le rapport de l'acide o-acétylsalicylique à l'acide aminé dans la solution réactionnelle a une valeur de 1:1,05 à 1:1,2.

10. Procédé suivant l'une des revendications 7 à 9, **caractérisé en ce qu'**un excès de 30 à 40 % d'acétone est ajouté.

11. Procédé suivant l'une des revendications 7 à 10, **caractérisé en ce que** la température pendant et après l'addition d'acétone est de 0°C.

12. Procédé suivant l'une des revendications 7 à 11, **caractérisé en ce qu'**on agite pendant 1 à 8 heures après l'addition d'acétone.

13. Procédé suivant l'une des revendications 7 à 12, **caractérisé en ce que** l'énergie d'agitation est de 0,04 à 0,06 W par litre de milieu réactionnel.

14. Procédé suivant l'une des revendications 7 à 13, **caractérisé en ce que** le séchage est effectué à une température inférieure à 35°C.

15. Procédé suivant l'une des revendications 7 à 14, **caractérisé en ce que** le séchage est effectué à une pression inférieure à 30 mbar.

16. Procédé suivant l'une des revendications 7 à 15, **caractérisé en ce qu'**on ajoute 10 % en poids de glycine.

17. Procédé suivant l'une des revendications 7 à 16, **caractérisé en ce qu'**il est mis en oeuvre dans des conditions stériles.

18. Médicament contenant au moins une composition suivant l'une des revendications 1 à 6 précédentes.

19. Médicament suivant la revendication 18, **caractérisé en ce qu'**il contient une ou plusieurs autres substances pharmaceutiques actives, en particulier un ou plusieurs antagonistes des récepteurs de ADP, antagonistes des récepteurs de GPIIb/IIIa, inhibiteurs de phosphodiestérase, antagonistes des récepteurs de thrombine, inhibiteurs du facteur Xa, antagonistes des récepteurs de HMG-CoA et/ou antagonistes du calcium.

20. Utilisation de compositions suivant l'une des revendications 1 à 6 précédentes pour la préparation d'un médicament destiné au traitement de l'arthrite, de névralgies, de myalgies et/ou de la migraine.

21. Utilisation de compositions suivant l'une des revendications 1 à 6 précédentes pour la préparation d'un médicament destiné au traitement de l'infarctus du myocarde, de l'apoplexie cérébrale, de maladies cardiaques ischémiques, de l'angine de poitrine, d'opérations de pontage, de l'angioplastie coronaire transluminale percutanée et/ou de l'implantation de stents.

22. Utilisation de compositions suivant l'une des revendications 1 à 6 précédentes pour la préparation d'une forme d'administration parentérale.

23. Utilisation de compositions suivant l'une des revendications 1 à 6 précédentes pour l'obtention de préparations à injecter et à perfuser sous forme de solutions, suspensions, émulsions, lyophilisats et poudres stériles.

24. Utilisation de compositions suivant l'une des revendications 1 à 6 précédentes pour la préparation d'un médicament destiné à l'administration intraveineuse, intra-artérielle, intracardiaque, intraspinale, intralombaire, intramusculaire, sous-cutanée, intracutanée ou intrapéritonéale.

25. Médicament selon la revendication 18, **caractérisé en ce qu'**il s'agit d'une forme d'application parentérale.

26. Médicament selon la revendication 18, **caractérisé en ce qu'**il s'agit de préparations à injecter et à perfuser sous forme de solutions, suspensions, émulsions, lyophilisats et poudres stériles.

27. Médicament selon la revendication 18, pour administration intraveineuse, intra-artérielle, intracardiaque, intraspinale, intralombaire, intramusculaire, sous-cutanée, intracutanée ou intrapéritonéale.
